# EUROPEAN PATENT SPECIFICATION

(11) **EP 3 021 817 B1**
(45) Date of publication and mention of the grant of the patent: **28.12.2016**
(21) Application number: 14733204.3
(22) Date of filing: 27.06.2014
(51) Int. Cl.: A61K 8/24, A61Q 11/00, A61K 8/19

(54) **REMINERALISING ORAL CARE PRODUCTS**
REMINERALISIERENDE MUNDPFLEGEPRODUKTE
PRODUITS D'HYGIÈNE ORALE DE REMINÉRALISATION

(30) Priority: 19.07.2013 EP 13177296
(43) Date of publication of application: 25.05.2016
(73) Proprietor: Unilever PLC, London, Greater London EC4Y 0DY (GB); Unilever N.V., 3013 AL Rotterdam (NL)
(72) Inventor: BRIGNOLI, Cinzia, I-20135 Milan (IT); HORNBY, Kate, Merseyside CH63 3JW (GB); JOINER, Andrew, Merseyside CH63 3JW (GB); PHILPOTTS, Carole Jane, Merseyside CH63 3JW (GB)
(74) Representative: Tansley, Sally Elizabeth
(86) International application number: PCT/EP2014/063706
(87) International publication number: WO 2015/007503

(56) References cited:
- WO-A1-2006/004991
- WO-A1-2008/068149
- GB-A- 1 516 525

## Description

### Field of the Invention

The invention relates to oral care products for the remineralisation of teeth. The oral care products comprise compositions that react together to form hydroxyapatite on the tooth surface, a material that can lead to remineralisation, repair and/or whitening of the teeth.

### Background of the Invention

The primary component of the enamel and dentin in teeth is calcium phosphate in the form of hydroxyapatite.

In the mouth, there is a natural equilibrium between hydroxyapatite being dissolved from the enamel of teeth and hydroxyapatite being formed on or in the teeth from substances occurring naturally in the saliva. This equilibrium is shifting continuously. Among other factors, it is determined by diet and physical condition.

If the equilibrium is such that hydroxyapatite is being dissolved, a cariogenic condition arises which is referred to as demineralisation. Exposure of the dental hard tissues to acid causes demineralisation, resulting in surface softening and a decrease in mineral density. Dental erosion (i.e. acid erosion or acid wear) is a surface phenomenon that involves demineralisation, and ultimately complete dissolution of the tooth surface by acids that are not of bacterial origin. Most commonly the acid will be of dietary origin. Due to today's lifestyles with increasing consumption of acidic drinks and foods, tooth erosion is becoming more prevalent and common.

If the equilibrium is such that hydroxyapatite is being formed, this is referred to as remineralisation. Remineralisation hardens and strengthens the teeth, thereby providing protection from and treatment for dental erosion and/or tooth wear. It also reduces the likelihood of tooth decay and improves the appearance of the teeth, in particular their whiteness. The teeth may also appear smoother and shinier as a result.

The presence of fluoride ions can enhance the natural remineralisation process and this is one of the accepted mechanisms by which fluoride toothpastes and rinses protect against caries. Fluoride is most effective during the developmental years from childhood to young adulthood.

However, the ability of fluoride to promote remineralisation can be limited by the availability of calcium and phosphate ions in saliva. Accordingly, oral products utilising calcium and phosphate ions have been designed.

US 5,605,675 discloses a process for remineralisation of dental enamel by application of a two-phase composition; one phase containing a water-soluble calcium compound and one phase containing a water soluble inorganic phosphate and a water-soluble fluorine compound.

US 4,083,955 discloses a process for remineralisation of dental enamel by sequential application of two compositions, the first comprising calcium ions and second comprising phosphate ions, or vice versa.

GB 1,408,922 suggests a two-compartment tube, the first being filled with a toothpaste containing calcium chloride and the second containing disodium hydrogen phosphate. Upon dispensing, the compositions are mixed thereby causing the precipitation of calcium phosphate onto the teeth.

WO98/07448 discloses a dual toothpaste comprising two separated compositions, one of them comprising a calcium salt (calcium gluconate or calcium lactate), the other one comprising a phosphate salt.

EP 2 089 040 describes an improved tooth remineralising method involving the delivery of an insoluble calcium salt (such as calcium silicate) to the surface of the teeth and converting this salt into hydroxyapatite in situ by the simultaneous or sequential application of a source of phosphate ions. The insoluble calcium salt and the phosphate ions are delivered from independent compositions. According to this publication, the use of an insoluble source of calcium ions enables the calcium to be deposited onto the teeth before premature interaction with phosphate in the saliva of the oral cavity can occur. Having deposited on the tooth enamel and/or dentin, slow reaction with the phosphate present in the saliva and the phosphate added with the second composition, results in hydroxyapatite being produced exactly where it is required.

It is an object of the present invention to provide an improved oral care product and method for building stronger, healthier teeth.

Another object of the present invention is to provide an oral care product and method for remineralising teeth which does not require excessively frequent product application or long treatment times and which can be accomplished by a consumer without intervention of a dentist.

### Summary of the Invention

The present invention provides an oral care product suitable for the remineralisation of teeth, the product comprising:
(i) a first composition having a non-aqueous liquid continuous phase including a thickening agent, a humectant, and an agent for the remineralisation of teeth; the agent for the remineralisation of teeth being a mixture of a calcium source and a phosphate source which, when delivered to the teeth results in the in situ generation of hydroxyapatite on teeth;
(ii) a second composition having an aqueous liquid continuous phase including a thickening agent and a humectant, the second composition comprising at least 30% by weight water (by weight based on the total weight of the second composition), and less than 2.5% by weight of phosphate source (by weight based on the total weight of the second composition);
in which the second composition is stored separately from the first composition prior to usage of the product in a manner to avoid contact between the water in the second composition and the agent for the remineralisation of teeth in the first composition;
and also in which the first and second compositions are adapted to be admixed and applied to the surface of the teeth for sustained contact.

### Detailed Description of the Invention

The term "remineralisation" in the context of the present invention means the in situ generation of hydroxyapatite on teeth.

### First Composition

The first composition of the product of the invention has a non-aqueous liquid continuous phase including, inter alia, an agent for the remineralisation of teeth which is a mixture of a calcium silicate and a phosphate source which, when delivered to the teeth results in the in situ generation of hydroxyapatite on teeth.

The amount of calcium silicate in the first composition according to the invention typically ranges from 1 to 30%, preferably from 5 to 20% by total weight calcium source based on the total weight of the first composition.

Illustrative examples of the types of phosphate source that may be used in this context include, for example, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the phosphate source is a mixture of trisodium phosphate and sodium dihydrogen phosphate.

The amount of phosphate source(s) (e.g. trisodium phosphate and sodium dihydrogen phosphate) in the first composition according to the invention typically ranges from 2 to 15%, preferably from 4 to 10% by total weight remineralising phosphate source based on the total weight of the oral care composition.

Mixtures of any of the above described materials may also be used.

The first composition according to the invention is non-aqueous. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than about 5%, more preferably no greater than about 3% by weight based on the total weight of the first composition.

The liquid continuous phase of the first composition of the invention comprises a thickening agent. The thickening agent helps to impart a desirable viscosity profile and desirable flow characteristics to the composition.

Suitable thickening agents for use in the first composition according to the invention are those which are operable in non-aqueous systems. Examples of such materials include organic macromolecules which do not necessarily require hydration with water in order to build viscosity, but can also build viscosity by alternative mechanisms such as by hydrogen bonding in the presence of hydroxyl donors such as polyols. Carboxyvinyl polymers have been found to be useful in this context. Preferred carboxyvinyl polymers for use in the invention have a molecular weight of at least about 750,000, more preferably at least about 1,250,000, most preferably at least about 3,000,000 g/mol. A suitable chemical class of such carboxyvinyl polymers for use in the invention includes crosslinked polymers having a polymer backbone derived from acrylic acid, substituted acrylic acid, or salts or esters thereof, in which the crosslinking agent contains two or more carbon-carbon double bonds and is derived from a polyhydric alcohol. Specific examples of such materials are homopolymers of acrylic acid cross-linked with allyl ethers of sucrose or pentaerythritol and homopolymers of acrylic acid cross-linked with divinyl glycol. An example of a suitable carboxyvinyl polymer for use in the compositions of the present invention is a homopolymer of acrylic acid crosslinked with allyl ethers of pentaerythritol, which is slightly pre-neutralised (1 to 3%) by potassium salt. This pre-neutralisation is done in order to precipitate polyacrylic acid in the presence of the polymerisation solvent. Such a material is commercially available, for example, as CARBOPOL® 974P NF Polymer, ex Lubrizol Advanced Materials, Inc. For ease of processing, the most preferred carboxyvinyl polymer for use in the compositions of the present invention is a homopolymer of acrylic acid crosslinked with pentaerythritol triallylether. Such a material is commercially available, for example, as SYNTHALEN® KP, ex 3V Sigma. Mixtures of any of the above described materials may also be used.

The level of thickening agent will depend on the particular type chosen, but generally ranges from 0.01 to 10% by total weight thickener based on the total weight of the first composition. When the thickening agent is a carboxyvinyl polymer (as described above), the amount of carboxyvinyl polymer in the first composition suitably ranges from 0.01 to 5%, preferably from 0.02 to 1%, more preferably from 0.05 to 0.25% by total weight carboxyvinyl polymer based on the total weight of the first composition.

The liquid continuous phase of the first composition of the invention comprises a humectant. The humectant helps to keep the composition from hardening or crystallizing upon exposure to air. It also helps to give the composition a moist feel to the mouth, and may in some cases impart a desirable sweetness.

Preferred humectants for use in the invention include organic polyols having 3 or more hydroxyl groups in the molecule (hereinafter termed "organic polyols"). Examples of such materials include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The level of humectant will depend on the particular type chosen, but generally ranges from about 20 to 90% by weight based on the total weight of the first composition. When the humectant is one or more organic polyols (as described above), the amount of organic polyol suitably ranges from 35 to 75%, more preferably from 45 to 70% by total weight organic polyol based on the total weight of the composition. Most preferably the first composition of the invention is organic polyol-based.

In the context of the present invention, the term "organic polyol-based" means that the composition is not oil-based or water-based, but instead, organic polyols (as defined above) are a principal component in the composition. By "principal component" is meant that the organic polyols (as defined above) when taken together, make up a higher portion of the first composition's weight than any other compound. Ideally the first composition of the invention is glycerol-based (i.e. glycerol makes up a higher portion of the composition's weight than any other compound) and contains from 45 to 70% by weight glycerol based on the total weight of the first composition.

The first composition of the invention is typically in the form of an extrudable semi-solid such as a cream, paste or gel (or mixture thereof).

The viscosity of the first composition of the invention generally ranges from 10,000 to 100,000 cps, and preferably ranges from 30,000 to 60,000 cps, (when measured at 25°C on a Brookfield viscometer).

The pH of the first composition of the invention generally ranges from 7 to 10.5, and preferably ranges from 8 to 9, when measured at 25° C using conventional pH sensitive electrodes.

The first composition according to the invention may optionally include further ingredients to enhance performance and/or consumer acceptability.

For example, the first composition may include abrasive amorphous silica particles which have a weight mean particle size (d50) ranging from 3 to 15 microns.

Preferred abrasive amorphous silica particles for use in the invention have a weight mean particle size in the range 3 to 6 microns. The weight mean particle size of the silica may suitably be determined using a Malvern Mastersizer® model S, with a 300 RF lens and MS 17 sample presentation unit. This instrument, made by Malvern Instruments, Malvern, Worcestershire, uses the principle of Fraunhofer diffraction, utilising a low power He/Ne laser. Before measurement, the sample is dispersed ultrasonically in water for 5 minutes to form an aqueous suspension. The Malvern Mastersizer® measures the weight particle size distribution of the silica. The weight mean particle size (d50) or 50 percentile and the percentage of material below any specified size are easily obtained from the data generated by the instrument.

Preferably, the abrasive amorphous silica particles employed are precipitated silica.

Suitable precipitated silicas for use as abrasive amorphous silica particles in the invention are commercially available and include those marketed by PQ Corporation under the trade names SORBOSIL® AC 43 and SORBOSIL® AC 33.

Mixtures of any of the above described materials may also be used.

The level of abrasive amorphous silica particles (as defined above) generally ranges from 0.05 to 5%, preferably from 0.1 to 3%, more preferably from 0.2 to 0.8%, by total weight abrasive amorphous silica particles (as defined above) based on the total weight of the first composition.

The first composition may also include one or more surfactants. Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines.

Mixtures of any of the above described materials may also be used.

The level of surfactant generally ranges from 0.2 to 5% by total weight surfactant based on the total weight of the first composition.

The first composition may also contain one or more liquid polyethylene glycols having a melting point below 25°C. Preferably the melting point ranges from -50 to 22°C, more preferably from -15 to 8° C, most preferably from 4 to 8°C.

The liquid polyethylene glycol(s) helps to make processing of the composition easier, especially at high shear, by reducing the overall viscosity of the liquid continuous phase.

Preferred liquid polyethylene glycols have an average value of n in the general formula H(OCH2CH2)n OH (as described above) ranging from about 4 to 12, more preferably about 6 to 8, most preferably about 8. The average molecular weight suitably ranges from about 190 to 630, more preferably from about 285 to 420, most preferably from about 380 to 420 g/mol. Suitable commercially available materials include for example PEG 400 (ex BDH Chemicals). Mixtures of any of the above described materials may also be used.

The amount of liquid polyethylene glycol (as defined above) suitably ranges from 1 to 20%, preferably from 5 to 15%, more preferably from 8 to 12% by total weight liquid polyethylene glycol (as defined above) based on the total weight of the first composition.

The first composition may also include one or more solid particulate polyethylene glycols having a melting point of 25°C or above. Preferably the melting point ranges from 35 to 65°C, more preferably from 55 to 60° C.

Polyethylene glycols have the general formula H(OCH2CH2)n OH, where n is the number of repeating oxyethylene units. Commercially available polyethylene glycols are usually not uniform chemical compounds, but instead consist of a distribution of similar polymer members of the homologous polyethylene glycol series, defined by average values of n and molecular weight. The melting point generally increases with increasing average values of n and molecular weight. Suitable solid polyethylene glycols have an average value of n in the above general formula ranging from about 20 to 220, preferably from about 40 to 150, more preferably from about 32 to 90, most preferably from about 60 to 75. The average molecular weight suitably ranges from about 950 to 11,250, preferably from about 1800 to 6600, more preferably from about 1400 to 4400, most preferably from about 2700 to 3700 g/mol. Suitable commercially available materials include for example Polyglykol® 3000 (ex Clariant). Mixtures of any of the above described materials may also be used.

The amount of solid polyethylene glycol (as defined above) suitably ranges from 0.1 to 5%, preferably from 0.5 to 3%, more preferably from 1 to 2.5% by total weight solid polyethylene glycol (as defined above) based on the total weight of the first composition.

The particles of solid polyethylene glycol(s) help to provide an optimised microstructure for the composition, particularly when the composition is prepared by a process route involving carefully controlled heating and cooling to form crystals of the solid polyethylene glycol. An example of such a process route comprises the following steps:
(i) forming a mixture of humectant (e.g. glycerol) and liquid polyethylene glycol (e.g. PEG400);
(ii) heating the mixture to a temperature above the melting point of the solid polyethylene glycol (e.g. PEG3000) and dispersing the solid polyethylene glycol into the mixture;
(iii) adding powdered ingredients (such as abrasive cleaning agent and/or surfactant) to the mixture so obtained;
(iv) adding the thickening agent to the mixture so obtained, and
(v) cooling the mixture to form crystals of the solid polyethylene glycol(s).

Being non-aqueous, the first composition according to the invention is particularly suitable as a vehicle for oral care actives which may be physically or chemically incompatible with water, or which may function less efficiently in an aqueous environment.

Specific examples of oral care actives which may be included in the first composition include:
fluoride sources such as sodium fluoride, stannous fluoride, sodium monofluorophosphate, zinc ammonium fluoride, tin ammonium fluoride, calcium fluoride, cobalt ammonium fluoride and mixtures thereof;
plant-derivable antioxidants such as flavonoid, catechin, polyphenol, and tannin compounds and mixtures thereof;
antioxidant vitamins such as tocopherols and/or derivatives thereof, ascorbic acid and/or derivatives thereof and mixtures thereof.

The first composition may also contain further optional ingredients customary in the art such as buffers, flavouring agents, sweetening agents, colouring agents, opacifying agents, preservatives and the like.

### Second Composition

The second composition of the product of the invention has an aqueous liquid continuous phase including inter alia a thickening agent and a humectant.

Suitable thickening agents for use in the second composition according to the invention are those which are operable in aqueous systems. Examples of such materials include cellulose based gums.

A preferred class of cellulose based gum for use in the second composition according to the invention are cellulose ethers.

Specific examples of such materials include: carboxyalkyl cellulose ethers such as carboxyethyl cellulose and carboxymethyl cellulose (CMC); mixed ethers such as carboxyalkyl hydroxyalkyl ethers, for example carboxymethyl hydroxyethyl cellulose (CMHEC); hydroxyalkyl celluloses such as hydroxyethyl cellulose and hydroxypropyl cellulose; alkylhydroxyalkyl celluloses such as methylhydroxypropyl cellulose; alkyl celluloses such as methyl cellulose, ethyl cellulose, and propyl cellulose; alkylcarboxyalkyl celluloses such as ethylcarboxymethyl cellulose; alkylalkyl celluloses such as methylethyl cellulose; and hydroxyalkylalkyl celluloses such as hydroxypropylmethyl cellulose. The above cellulose ethers are generally available commercially in various grades. The carboxy-substituted cellulose ethers are available as the alkali metal salt, usually the sodium salt (SCMC). SCMC is commercially available in various degrees of carboxylate substitution ranging from about 0.3 up to the maximum degree of substitution of 3.0. Preferred sodium carboxymethyl celluloses (SCMCs) include those with a degree of substitution of from 0.65 to 0.95, more preferably from 0.85 to 0.95, and a viscosity ranging from 250 to 10000 mPa.s, more preferably from 1,000 to 6,000 mPa.s (when measured as a 1% solution in distilled water at 25°C on a Brookfield viscometer).

The level of thickening agent will depend on the particular type chosen, but generally ranges from 0.01 to 10% by total weight thickener based on the total weight of the second composition. When the thickening agent is a cellulose ether (as described above), the amount of cellulose ether in the second composition suitably ranges from 0.01 to 5%, preferably from 0.02 to 1%, more preferably from 0.5 to 1.5% by total weight cellulose ether based on the total weight of the second composition.

Suitable humectants for use in the second composition according to the invention include organic polyols having 3 or more hydroxyl groups in the molecule (as described above in relation to the first composition and hereinafter termed "organic polyols"). Examples of such materials include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The level of humectant will depend on the particular type chosen, but generally ranges from about 20 to 90% by weight based on the total weight of the second composition. When the humectant is one or more organic polyols (as described above), the amount of organic polyol suitably ranges from 30 to 65%, more preferably from 45 to 50% by total weight organic polyol based on the total weight of the second composition.

The second composition according to the invention comprises at least 30% by weight water (by weight based on the total weight of the second composition). Preferably the level of water ranges from 50 to 65%, more preferably from 50 to 55% by weight based on the total weight of the second composition.

The second composition according to the invention comprises less than 2.5% by weight of phosphate source (by weight based on the total weight of the second composition). Specific examples of phosphate sources according to the invention are described above in relation to the first composition.

Preferably, the second composition is substantially free of phosphate source. The term "substantially free" in this context generally means that the content of phosphate source is less than 0.1%, more preferably less than 0.01%, and most preferably less than 0.001% by weight based on the total weight of the second composition.

The second composition of the invention is typically in the form of an extrudable semi-solid such as a cream, paste or gel (or mixture thereof).

The viscosity of the second composition of the invention generally ranges from 1,000 to 30,000 cps, and preferably ranges from 10,000 to 20,000 cps, when measured at 25°C on a Brookfield viscometer.

The pH of the second composition of the invention generally ranges from 7 to 9, and preferably ranges from 7.5 to 8.5, when measured at 25° C using conventional pH sensitive electrodes.

The second composition according to the invention may optionally include further ingredients to enhance performance and/or consumer acceptability.

Preferably the second composition includes a fluoride source such as sodium fluoride, stannous fluoride, sodium monofluorophosphate, zinc ammonium fluoride, tin ammonium fluoride, calcium fluoride, cobalt ammonium fluoride and mixtures thereof.

The second composition may also contain further optional ingredients customary in the art such as buffers, flavouring agents, sweetening agents, colouring agents, opacifying agents, preservatives and the like.

### Product Form and Packaging

In the oral care product of the invention, the second composition is stored separately from the first composition prior to usage of the product in a manner to avoid contact between the water in the second composition and the agent for the remineralisation of teeth in the first composition.

In packaging the oral care product of the invention, any convenient means for effecting the separation of the first composition from the second composition before use may be used. For example, a single container may be compartmentalized so that the first composition and the second composition are housed in separate compartments and are dispensed synchronously as a ribbon, and admixed prior to application on the teeth. Alternatively, the first composition and the second composition may be housed in physically separate containers (e.g. tubes) from which they are dispensed for admixture just prior to use.

### Mode of Use

In the oral care product of the invention, the first and second compositions are adapted to be admixed and applied to the surface of the teeth for sustained contact.

Accordingly, the invention also provides a method of remineralising teeth, in which the oral care product as described above is applied to the surface of the teeth for sustained contact.

In the context of the present invention sustained contact means the product is left on the teeth for 1 to 10 minutes, preferably about 2 to 5 minutes before being removed, before being removed by brushing or rinsing.

For example, separate ribbons of the first and second compositions respectively may be extruded sequentially onto the bristles of a toothbrush, which is then agitated against the surface of the teeth.

However, it is preferred that the first and second compositions are applied to the surface of the teeth by means of an applicator device which is adapted to be placed in the mouth.

A preferred form of applicator device in this context comprises a front wall, the inner face of which is adapted in use to contact the front face of the teeth, a bridge portion which is adapted in use to contact the biting edge of the teeth and a rear wall, the inner face of which is adapted in use to contact the rear face of the teeth.

The applicator device may take various shapes to accommodate the dental arch of the consumer but in a preferred arrangement the device is generally U-shaped defining a U-shaped channel for receiving the dental arch.

In order to allow adjustment to suit various shapes of mouth, the device may be formed of flexible, plastically deformable material so that it may be adjusted in shape to suit the consumer's mouth. Ideally the device is formed of a thermoplastic material that may be softened in hot water and moulded to the consumer's teeth prior to use.

In a typical mode of use of the oral care product of the invention, the first and second compositions are admixed and applied to the surface of the teeth by means of an applicator device such as described above.

Preferably the first and second compositions are dispensed into the applicator device, (typically into the U-shaped channel for receiving the dental arch as described above) and then admixed. The applicator device is then placed into the mouth to enable sustained contact of the first and second compositions with the surface of the teeth.

Following such application, the device containing the first and second compositions is typically left on the teeth for several minutes before being removed. Preferably the device is left on the teeth for about 1 to 10 minutes, more preferably about 2 to 5 minutes before being removed.

Preferably the application of the oral care product of the invention is carried out on a daily basis. More preferably the application of the oral care product of the invention is carried out once daily for a period of several consecutive days, in addition to a regular regime of tooth brushing (preferably at least twice daily).

More preferably the application of the oral care product of the invention is carried out once daily for a period of 3 to 5 consecutive days, in addition to at least twice-daily tooth brushing with a remineralising toothpaste. Most preferably the remineralising toothpaste is a calcium silicate toothpaste such as is described in WO2011/160996.

The invention is further illustrated with reference to the following, non-limiting Example.

### EXAMPLE

The following formulation illustrates a first composition of an oral care product according to the invention:

| Ingredient | (wt%) |
|---|---|
| Glycerol (99.5% a.i.) | 59.94 |
| PEG 400 | 10.5 |
| PEG 3000 | 2 |
| Timiron® Ice Crystal | 0.5 |
| Sodium monofluorophosphate | 1.11 |
| Trisodium phosphate anhydrous | 3.8 |
| Monosodium dihydrogen phosphate anhydrous | 3.2 |
| Calcium silicate | 15 |
| Sodium saccharin | 0.25 |
| Sodium lauryl sulphate | 2.00 |
| SORBOSIL®AC43 (ex PQ Corp.) | 0.50 |
| SYNTHALEN®KP (ex 3V Sigma) | 0.10 |
| Flavour | 1.10 |

The following formulation illustrates a second composition of an oral care product according to the invention:

| Ingredient | (wt%) |
|---|---|
| Glycerol (99.5% a.i.) | 47.6795 |
| Water | 50 |
| SCMC9H (sodium carboxymethylcellulose) | 1 |
| Benzyl alcohol | 0.3 |
| Phenoxyethanol | 0.3 |
| Sodium fluoride | 0.32 |
| Colour | 0.0005 |
| Ethylhexylglycerin | 0.3 |

The product is used as follows:

The product is applied to the teeth via a gum shield shaped applicator formed from thermoplastic material.

In a first stage, the empty gum shield is soaked in hot water for 5 seconds and then manually moulded onto the surfaces of the teeth so that it is shaped to fit.

After removal of the gum shield from the mouth, portions of the first and the second compositions respectively are dispensed into its interior. The two compositions are mixed inside the gum shield to obtain a homogeneous mix.

The filled gum shield is put onto the teeth and held in the mouth for 3 minutes followed by removal and rinsing.

The product of the invention is able to effect complete repair of acid eroded enamel, when used for 3 consecutive days in addition to a regular regime of tooth brushing with a remineralising toothpaste containing calcium silicate.

## Claims

1. An oral care product suitable for the remineralisation of teeth, the product comprising:
(i) a first composition having a non-aqueous liquid continuous phase including a thickening agent, a humectant, and an agent for the remineralisation of teeth; the agent for the remineralisation of teeth being a mixture of a calcium silicate and a phosphate source which, when delivered to the teeth results in the in situ generation of hydroxyapatite on teeth;
(ii) a second composition having an aqueous liquid continuous phase including a thickening agent and a humectant, the second composition comprising at least 30% by weight water (by weight based on the total weight of the second composition), and less than 2.5% by weight of phosphate source (by weight based on the total weight of the second composition);
in which the second composition is stored separately from the first composition prior to usage of the product in a manner to avoid contact between the water in the second composition and the agent for the remineralisation of teeth in the first composition;
and also in which the first and second compositions are adapted to be admixed and applied to the surface of the teeth for sustained contact.

2. An oral care product according to claim 1, in which the phosphate source is a mixture of trisodium phosphate and sodium dihydrogen phosphate.

3. An oral care product according to claim 1 or claim 2, in which the thickening agent in the first composition is a carboxyvinyl polymer.

4. An oral care product according to any preceding claim, in which the thickening agent in the second composition is a cellulose ether.

5. A method of remineralising teeth, in which an oral care product as described in any one of claims 1 to 4 is applied to the surface of the teeth by means of an applicator device which is adapted to be placed in the mouth.

6. A method according to claim 5, in which the applicator device comprises a front wall, the inner face of which is adapted in use to contact the front face of the teeth, a bridge portion which is adapted in use to contact the biting edge of the teeth and a rear wall, the inner face of which is adapted in use to contact the rear face of the teeth.

## Patentansprüche

1. Mundpflegeprodukt mit Eignung zum Remineralisieren von Zähnen, wobei das Produkt umfasst:
(i) eine erste Zusammensetzung mit einer nicht-wässrigen flüssigen kontinuierlichen Phase, die ein Verdickungsmittel, ein Feuchthaltemittel und ein Mittel zum Remineralisieren von Zähnen enthält,
wobei das Mittel zum Remineralisieren von Zähnen eine Mischung eines Calciumsilikats und einer Phosphatquelle darstellt, die, wenn sie auf die Zähne aufgebracht wird, auf den Zähnen zu einer in situ-Erzeugung von Hxydroxyapatit führt,
(ii) eine zweite Zusammensetzung mit einer wässrigen flüssigen kontinuierlichen Phase, die ein Verdickungsmittel und ein Feuchthaltemittel enthält, wobei die zweite Zusammensetzung mindestens 30 Gewichts-% Wasser (in Gewicht, bezogen auf das Gesamtgewicht der zweiten Zusammensetzung) und weniger als 2,5 Gewichts-% Phosphatquelle (in Gewicht, bezogen auf das Gesamtgewicht der zweiten Zusammensetzung) enthält,
in welchem die zweite Zusammensetzung von der ersten Zusammensetzung vor Verwendung des Produkt derart separat gelagert wird, dass der Kontakt zwischen dem Wasser in der zweiten Zusammensetzung und dem Mittel zum Remineralisieren der Zähne in der ersten Zusammensetzung vermieden wird, und in welchem ferner die ersten und die zweiten Zusammensetzungen zum Vermischen und zum Auftragen auf die Oberfläche der Zähne für einen fortdauernden Kontakt angepasst sind.

2. Mundpflegeprodukt nach Anspruch 1, in welchem die Phosphatquelle eine Mischung von Trinatriumphosphat und Natriumdihydrogenphosphat darstellt.

3. Mundpflegeprodukt nach Anspruch 1 oder Anspruch 2, in welchem das Verdickungsmittel in der ersten Zusammensetzung ein Carboxyvinylpolymer darstellt.

4. Mundpflegeprodukt nach irgendeinem vorhergehenden Anspruch, in dem das Verdickungsmittel in der zweiten Zusammensetzung Celluloseether darstellt.

5. Verfahren zum Remineralisieren von Zähnen, bei welchem ein Mundpflegeprodukt, wie in irgendeinem der Ansprüche 1 bis 4 beschrieben, mittels einer Auftragsvorrichtung, die geeignet ist, in den Mund gesetzt zu werden, auf die Oberfläche von Zähnen aufgetragen wird.

6. Verfahren nach Anspruch 5, in welchem die Auftragsvorrichtung eine Vorderwand, wobei deren Innenfläche im Gebrauch geeignet ist, die Vorderfläche der Zähne zu berühren, einen Brückenabschnitt, der bei Verwendung geeignet ist, die Beißkante der Zähne zu berühren, und eine Rückwand, wobei deren Innenfläche bei Verwendung geeignet ist, die Rückfläche der Zähne zu berühren, umfasst.

## Revendications

1. Produit pour le soin oral approprié pour la reminéralisation des dents, le produit comprenant :
(i) une première composition présentant une phase continue liquide non aqueuse incluant un agent épaississant, un humectant, et un agent pour la reminéralisation des dents ;
l'agent pour la reminéralisation des dents étant un mélange d'un silicate de calcium et d'une source de phosphate qui, lorsqu'il est délivré aux dents, résulte en la production in situ d'hydroxyapatite sur les dents ;
(ii) une seconde composition présentant une phase continue liquide aqueuse incluant un agent épaississant et un humectant, la seconde composition comprenant au moins 30 % en masse d'eau (en masse rapporté à la masse totale de la seconde composition), et moins de 2,5 % en masse de source de phosphate (en masse rapporté à la masse totale de la seconde composition) ;
dans lequel la seconde composition est stockée séparément de la première composition avant l'utilisation du produit de manière à éviter un contact entre l'eau dans la seconde composition et l'agent pour la reminéralisation des dents dans la première composition ;
et également dans lequel les première et seconde compositions sont adaptées pour être mélangées et appliquées à la surface des dents pour un contact prolongé.

2. Produit pour le soin oral selon la revendication 1, dans lequel la source de phosphate est un mélange de phosphate de trisodium et de dihydrogénophosphate de sodium.

3. Produit pour le soin oral selon la revendication 1 ou la revendication 2, dans lequel l'agent épaississant dans la première composition est un polymère carboxyvinylique.

4. Produit pour le soin oral selon l'une quelconque des revendications précédentes, dans lequel l'agent épaississant dans la seconde composition est un éther de cellulose.

5. Procédé de reminéralisation des dents, dans lequel un produit pour le soin oral comme décrit dans l'une quelconque des revendications 1 à 4 est appliqué à la surface des dents au moyen d'un dispositif d'applicateur qui est adapté pour être placé dans la bouche.

6. Procédé selon la revendication 5, dans lequel le dispositif d'applicateur comprend une paroi avant, dont la face interne est adaptée lors d'une utilisation pour être en contact avec la face avant des dents, une portion de pont qui est adaptée lors d'une utilisation pour être en contact avec le bord de morsure des dents et une paroi arrière dont la surface interne est adaptée lors d'une utilisation pour être en contact avec la face arrière des dents.
